# EUROPEAN PATENT SPECIFICATION

(11) **EP 4 543 474 B1**
(45) Date of publication and mention of the grant of the patent: **11.11.2026**
(21) Application number: 23742412.2
(22) Date of filing: 23.06.2023
(51) Int. Cl.: A61K 36/15, A61P 19/02, A61K 36/324, A61K 36/48, A61K 36/77, A61K 47/46, A61K 47/54, A61K 47/69, A61P 21/00, A61K 9/00, A61K 9/107

(54) **COMPOSITION FOR TOPICAL USE FOR PAINFUL AND INFLAMMATORY STATES**
ZUSAMMENSETZUNG ZUR TOPISCHEN ANWENDUNG FÜR SCHMERZHAFTE UND ENTZÜNDLICHE ZUSTÄNDE
COMPOSITION À USAGE TOPIQUE POUR ÉTATS DOULOUREUX ET INFLAMMATOIRES

(30) Priority: 27.06.2022 IT 202200013468
(43) Date of publication of application: 30.04.2025
(73) Proprietor: Prodeco Pharma S.r.l., 31033 Castelfranco Veneto (TV) (IT)
(72) Inventor: CATANIA, Alberto, 31033 Castelfranco Veneto TV (IT); GIANESIN, Ketty, 31033 Castelfranco Veneto TV (IT); LUCCHESE, Paolo, 31033 Castelfranco Veneto TV (IT)
(74) Representative: Di Giovine, Paolo
(86) International application number: PCT/IB2023/056505
(87) International publication number: WO 2024/003686

(56) References cited:
- EP-A1- 2 149 378
- EP-A1- 2 702 984
- EP-A1- 3 795 146
- WO-A1-2015/000064
- US-A1- 2016 095 890
- US-A1- 2021 322 366
- HÜSCH JAN ET AL: "Enhanced absorption of boswellic acids by a lecithin delivery form (Phytosome ) ofBoswelliaextract", FITOTERAPIA, IDB HOLDING, MILAN, IT, vol. 84, 23 October 2012 (2012-10-23), pages 89 - 98, XP028968965, ISSN: 0367-326X, DOI: 10.1016/J.FITOTE.2012.10.002

## Description

### FIELD OF THE INVENTION

The present invention relates to a composition for topical use comprising β-caryophyllene or oleoresin of *Copaifera officinalis* containing it, boswellic acids or resin extracts of *Boswellia serrata* containing it, and escin or extracts of *Aesculus hippocastanum* containing it, to offer relief in painful and inflammatory states of traumatic or rheumatic nature affecting joints, muscles, tendons and ligaments. The present invention further relates to said compositions for use in the treatment of painful and inflammatory states of traumatic or rheumatic nature.

### STATE OF ART

The musculoskeletal disorders are always associated to joint damages and pain. Consequently, they are defined painful musculoskeletal disorders. They include a great number of disorders, going from the peripheral joint diseases (such as rheumatic disease, rheumatoid arthritis, connective tissue diseases, osteoarthritis, gout, sarcoidosis and infectious arthritis) to myalgias (such as rheumatic polymyalgia, ankylosing spondylitis and psychogenic rheumatisms), or joint, neurological, degenerative, autoimmune, vascular and traumatic disorders which can involve joints, limbs, ankles and the lumbosacral region, by causing pain and functional impairment. The most common degenerative inflammatory disease of the skeletal muscle apparatus is osteoarthritis (OA), also known as degenerative disease of joints.

In case of OA, the most affected joints are the small joints of hands (first carpometacarpal joints, proximal and distal interphalangeal joints) and feet (metatarsophalangeal joints), as well as the joints of hip, knee and vertebral column. The main symptoms are the progressive pain, which at first is mild, but afterwards becomes constant and invalidating, the crunching of the affected joints, the joint deformity due to the increase in the bone tissue in the synovial liquid and to synovitis, the limited motions of the joint with spasms and joint instability. There could be even a valgus deformity of the knee. The disease has a chronic-degenerative course.

The most commonly used painkilling drugs are paracetamol, acetylsalicylic acid, FANS, indomethacin, methotrexate and cyclosporine A, which have serious side effects such as liver and kidney diseases, gastrointestinal ulcers and immunosuppression.

The same drugs are used for almost all painful rheumatic syndromes, especially in the rheumatoid arthritis, where the anatomopathological damage leads to clinical symptoms substantially similar to those of osteoarthritis.

The patent application EP3795146A1 discloses a pain-relieving and anti-inflammatory composition for local use.

In the state of art, then, the problem of providing methods and compositions capable of treating painful and inflammatory states of traumatic or rheumatic nature and relieving their symptoms, for example in arthritis, contractures, injuries and tendinitis, is still much felt. In particular, compositions based upon ingredients of natural origin which do not have the disadvantages of the known compositions.

### GLOSSARY

Copaiba oil *(Copaifera officinalis* (Jacq.) L.): the used plant portion is the oleoresin obtained from the trunk. The genus *Copaifera* comprises several tropical trees of the *Fabacee* family, especially known for the resins which they produce in quantity and which consist of an oil-resinous liquid (also called balm). Copaiba tree, very widespread in South America and Amazon, appears like a very branched tree, high up to 30 m with panicle inflorescences bearing many white flowers.

Traditionally used for whoever suffers from skin problems such as acne or psoriasis, thanks to its marked germicidal and anti-inflammatory properties; its antimicrobial effectiveness has been widely documented as from the XIX century and it was widely used to fight a whole series of infections until the discovery of penicillins. Moreover, the astringent effect makes it particularly suitable to normalize greasy skins, mixed skins and skins with acne tendency. In the Northern Brazilian populations, copaiba oil is also used as laxative, diuretic, anti-rheumatic drug, for the treatment of gonorrhea and even for fatigue and depression. Oleoresin is obtained from internal cavities of trunks, therefrom a sticky and viscous liquid is collected (called oleoresin) in which the main component is β-caryophyllene (up to 48%), but there are also other terpenes such as the copalic acid, α-copaene, α-humulene, α-bergamotene and deltacadinene.

### Nanosomal Copaiba oil:

Nanosome represents a delivery system based on nanovesicles (liposomes with sizes 150-300 nm) which allow to incapsulate an active principle with high effectiveness and to release then the incapsulated compound. Such technology allows to guarantee greater stability of the extract, to increment the bioavailability and the effectiveness thanks to its capability of crossing the biological membranes. In particular, a transportation system was developed capable of releasing the active principle in the deepest layers of epidermis to act more intensely and precise on the structures and cells present therein: and then keratinocytes, Langerhans' cells, melanocytes (cells actually expressing receptor CB2).

Boswellia serrata *(Boswellia serrata* Roxb. ex Colebr. *(Burseraceae* family)): the used plant portion is the gum resin obtained by incision of the bark. The collection is performed by producing oval decortications on the branches by using a kind of chisel sharpened on one side to decorticate the branches and not sharpened on the other side to collect resin. From a botanical point of view, it appears like a shrub of 3-6 m in height which grows in the mountainous, arid and wild areas. The feature of this kind of shrubs is to have a bark rich in resin channels therefrom a scented resin is obtained known under the name of incense. As far as the active components are concerned, it is a plant rich in oleoresins, that is mixtures of resins and essential oils. The resinous fraction mainly consists of triterpenes (55%). There is also a certain amount of gums and gum resins (23%).

Among the components of the oleo-resinous fraction there are:
- Monoterpenes;
- Sesquiterpenes;
- Lipophilic pentacyclic triterpenic acids (boswellic acids) of oleananic (α-boswellic acids) and ursanic (β-boswellic acids) type;
- Diterpenes (incense, incense acetate, cembrenol (serratol));
- Lipophilic pentacyclic triterpenic acids of lupanic type (lupeolic acid).

Phytosomal Boswellia: the subject extract is a solid dispersion of Boswellia extract in natural phospholipids (from soy). This type of formulation, said "phytosome", consists in a transport and release biomimetic system useful to improve the pharmacokinetic profile of the poorly bioavailable active components without having to recourse to the addition of pharmacological adjuvants or to structural modifications of the ingredients. The phytosome technology, apart from guaranteeing the extract stability, improves the bioavailability and effectiveness thereof thanks to its capability of crossing the biological membranes, rich in lipids. At skin level, the phytosome crosses the skin barrier by interacting with its structural components; once reached the dermis, it undergoes a slow and progressive decomplexation, by realising in situ the active components. With respect to the liposomes, they are characterized by a high ratio bioactive/lipidic phase with a stoichiometry of 1:1-1:3 between the adjuvant of active formulation and the phospholipidic one. In the subject extract the two compounds form a non-covalent adduct in ratio 1:1 by weight, then 1 portion of microcrystalline cellulose is added to improve formulation, with an overall content of total boswellic acids of at least 25%. In the phytosomes the active principle is anchored to the polar head of the phospholipide by becoming integrating portion thereof of micellar membrane; on the contrary, in the liposomes the active principle is contained inside the micellar structure formed by the phospholipides. Based upon its chemical-physical features the phytosome is to be meant as a complex which increases the biological activity of the non-phytosomal system, it improves the absorption thereof and allows a longer action duration.

Escin: escin is a mixture of triterpene saponins isolated from the Horse Chestnut seed *(Aesculus hippocastanum),* wherein usually they are present in an amount of 3-10%. These pentacyclic triterpene saponins were identified as protoescigenin and barringtogenol and the fraction was called (and subsequently β-escin). Escin includes a trisaccharide bound to the 3-OH residue (glucose, xylose and galactose) whereas the domains C21 and C22 are esterified with an organic acid (for example, angelic, tiglinic or acetic acid). The main isomers of escin are β-escin and kryptoescin. β-escin is relatively insoluble in water whereas kryptoescin is readily soluble in water, but considerably less active of β-escin. Escin has optimum anti-oedema properties which make it particularly useful both in case of trauma and in case of venous insufficiency, water retention and cellulite, thanks to its activity at level of capillaries. In particular, its action mechanism is based upon its capability of reducing the number and the diameter of the endothelial pores responsible for the physiological exchange of liquids between the blood vessels and the surrounding environment of connective tissue. The action at this level, by reducing the exit of interstitial liquids, generates the tissue resorption of oedema.

Phytosomal escin: escin in complexed form within a phytosome with β-sitosterol and/or with distearoylphosphatidylcholine. β-sitosterol is a sterol of plant origin, a molecule structurally similar to choresterol which differs in the presence of ethyl and methyl groups in the lateral chain. Then, like cholesterol, they are capable of stabilizing the phospholipidic bilayer of membrane by adjusting permeability and fluidity. Then, it follows that escin, when complexed with β-sitosterol and phospholipids is more tolerated by skin, more stable and more active thanks to the greater bioavailability which allows it to act longer.

Swiss stone pine essential oil *(Pinus cembra* L.): the used plant portions are the twigs. From the botanical point of view, it appears like a coniferous evergreen tree of genus *Pinus* living in the Alps, growing starting as from 1200 meters of altitude as far as the upper limit of subalpine coniferous forests, by finding optimum conditions between 1600 and 2100 m of altitude. This particular species differs from other pines in the coniferous leaves in bunches of 5: it is the only 5-needle pine present spontaneously in Europe. In therapeutic environment, the essential oil is used in case of cold, cough, sore throat, inhaled or oral bronchitis, as tea additive. It is traditionally used for toning and revitalizing the body in case of tiredness and physical weakness, apart from as soothing and relaxing remedy for treating pains and muscle tensions, neuropathic pain, joint pains, strains and small bruises. As far as the active components are concerned, the main constituents are monoterpenes, in particular there are: α-pinene (~45%), β-phellandrene (~15%), β-pinene (8.5%) and limonene (~5%). 103 different olfactive substances were identified as a whole.

Common comfrey *(Symphytum officinale* L.): the used plant portion is the root. Common comfrey is a perennial herbaceous plant, with pendulous flowers, of *Boraginaceae* family. It is a plant which can reach 130 cm in height, it has thick leaves and the roots succeed in extending into the ground as far as 180 cm in length. The floral stems, starting from half May, show bell flowers, with a colour ranging from white to mauve, depending upon the variety. As habitat it prefers swampy places, such as ditches, channels and bogs, but also wet meadows and forest edges. These plants can be found both in the hills and in the mountains up to an altitude of 1300 meters.

Traditional use: the name origin is Greek, *symphuo* and it means to weld, to join. The origin of this name is to be attributed to its use by folk medicine for the treatment of bone fractures, in fact it seems that it stimulates the formation of bone callus in case of fractures. Moreover, the plant is used still today to treat wounds and burns and to regenerate tissues after sprains, tears and strains. Compfrey was used even to treat cough, thanks to the presence of mucilages, however nowadays the use by oral route is not recommended in several countries (including Italy) due to the presence of some hepatotoxic molecules (pyrrolizidine alkaloids). Active components: the Compfrey root is particularly rich in allantoin (up to ~5%), mucilages (~30%), phenolic acids (cinnamic acid, chlorogenic acid, caffeic acid, rosmarinic acid), glyco-peptides and amino acids. Menthyl nicotinate and menthol: menthyl nicotinate, or menthylniconate, is a synthesis product of natural origin which conjugates the vasodilatory properties of the nicotinic acid with the refreshing ones of menthol. The menthyl nicotinate is capable of activating significantly the cutaneous microcirculation however without causing annoying hyperemia or irritation, on the contrary by providing a pleasant hot-cold sensory effect limited to the application area. In particular, the menthyl nicotinate derives from the chemical reaction between two natural compounds:
- the Menthol (obtained from the mint essential oil), having a refreshing and soothing effect on the skin;
- the Nicotinic acid also called Niacin or Vitamin B3 or Vitamin PP (Pellagra-Preventing), used by the organism to form coenzymes essential for the energy metabolism (NAD and NADP).

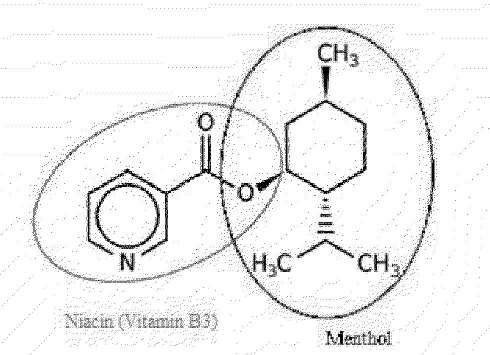

These coenzymes participate in more than 500 enzymatic reactions in the organism. The Nicotinic acid participates in the degradation and use of proteins, lipids and carbohydrates. It plays a fundamental role in the formation of red blood cells, in the blood circulation and in the oxygen transportation to the cells. Its properties are exploited in topical applications to improve the barrier effect, to improve oxygenation and nourishment of skin, to accelerate the cell differentiation and healing, as well as for its anti-inflammatory and detoxifying effects. The Menthyl nicotinate is easily absorbed by skin and hydrolyzed in its primary constituents: Menthol and Niacin. Since the menthol is a vasoconstrictor and Niacin is a vasodilator, few minutes after application there is a pleasant feeling of moderate freshness (due to the menthyl portion of the molecule) and a simultaneous feeling of stimulating warmth (due to the niacinic portion of the molecule): the result is a pleasant feeling of "cellular turgidity" or "hot-cold subcutaneous micromassage", indication of a beneficial increase in the microcirculatory activity, which can last up to an hour or more after application. The sensory activity of the menthyl nicotinate, as said previously, is mediated by the metabolites (menthol and niacin) which are released by hydrolysis thanks to the esterases present in the skin. In particular:
- the menthol activates the cold TRP receptors (TRPM8 and TRPA1) by increasing rapidly the intracellular calcium and by increasing the calcium flow through these channels, thus inducing sensory signals of "cold response" in the area surrounding the application;
- niacin, instead, apart from constituting a beneficial vitamin supply for the skin, activates the heat sensitive TRP receptor channels (TRPV1 and TRPV3), by decreasing the activation threshold temperature and thus inducing their activation already at the body temperature. The result of this simultaneous activation of the related TRP channels is a sensory transduction one of a kind, fresh and fizzy, which can be described as a "pleasant feeling of stimulating subcutaneous micromassage" ("fresh tingling"). Menthyl nicotinate actually constitutes the only molecule capable of activating simultaneously in the skin both the cold (TRPM8, TRPA1) and heat (TRPV1, TRPV3) thermoreceptors, thus providing a pleasant sensory, but composite and modulated, response. Thanks to the menthyl portion, menthyl nicotinate acts as a kind of "soothing", equilibrator and modulator of heat feeling, "by softening it" and extending it over time.

Eucalyptus essential oil *(Eucalyptus globulus* L.): the used plant portion is the leaf. From the botanical point of view, *Eucalyptus globulus* is a kind of evergreen tree plant, originally from Oceania and the Philippines, belonging to the *Myrtaceae* family. In the native Countries, the tree can reach 90 meters, in Italy instead its sizes stop at 25 meters. The stem has smooth bark, the flower is formed by a closed cup-shaped calyx that detaches upon flowering; the fruit is capsule-shaped with many small seeds inside. Eucalyptus essential oil is used in the traditional medicine for internal use in the treatment of asthma, fever, flu, whooping cough, loss of appetite, dyspeptic disorders, inflammatory and infectious diseases of kidneys and bladder, diabetes, rheumatic pains; externally it is used for wounds, acne, ulcers, stomatitis, gum bleeding, rheumatism, neuralgia, gonorrhea. Relatively to the active components, they constitute about 1.5-3% by weight of leaves; there are 1,8-cineole or eucalyptol (70-80%), and the main component (monoterpene), terpineol, α-pinene, alcohols and aliphatic aldehydes, alcohols and terpene ketones, euglobals (mono-and sesquiterpenic heterocyclic products), phenols and flavonoids (hyperoside, rutin and quercitrin).

Devil's claw *(Harpagophytum procumbens* L. (Burch.) DC.): the used plant portion is the root. From the botanical point of view, the plant is native to Southern and Eastern Africa, it can be frequently found in the South African plateau of Transvaal, in the desert and savannah areas. It appears like a perennial, climbing herbaceous plant with shoots originating from a tuberous root adhering to the ground. The leaves are erect, petiolate, fleshy and lobed. Single purple flowers are born from the leaf axils. The fruits are woody, jagged, provided with hooked thorns, which resemble claws. The analgesic and anti-inflammatory effect of harpagosides contained in the root are considered responsible for the analgesic and antipyretic effects of the plant, which justify the use thereof in case of situations which cause pain and inflammation such as tendinitis, osteoarthritis, rheumatoid arthritis, back pain, headache from cervical arthrosis, general neck pain, bruises, sciatica, arthritis, arthrosis. The active components are iridoid glucosides the main one thereof is arpagoside; triterpenes, flavonoids, phenolic acids, and polysaccharides are also present.

BIOSTERINE^{®}: is a phytocompound of Holy basil and Sage, titrated to 40% in Rosmarinic acid.

Holy basil *(Ocimum sanctum* L.): the used plant portion is grass. From the botanical point of view, the Holy basil è an annual herbaceous plant, belonging to the *Labiatae* family, erect with very branched stem, 30-60 cm in height, with simple green or purple leaves, decussate and petiolate. The lamina is lanceolate with a toothed margin. The flowers are purplish arranged in vertexes on an inflorescence. The Holy basil is native from South-tropical Africa, also widespread in the Southeast Asia and naturalized in Southern Europe, Australia and South America. The Holy basil has been appreciated as extraordinary health remedy since ancient times. In fact, it was used for treating cold, headache, disorders of the gastrointestinal tract, states of inflammation, heart diseases, various forms of poisoning and malaria. It is used even as tonic, anti-stress and considered an "elixir of life". It is used even in the Western phytotherapy and there are numerous studies proving its health-promoting activity.

As to the active components, they are alkaloids, fatty acids, carbohydrates, proteins, glycosides, phenols, saponins, tannins, terpenes. The main functional active components are mostly terpenes and volatile phenols: eugenol, caryophyllene, terpinene-4-olo, decylaldehyde, salinene, α-pinene, β-pinene, camphene, carvacrol, rosmarinic acid, terpen-ursolic acid, ursolic acid, juvocimene I and II, thymol, camphor, xanthomicrol, caffeate, myrcenol and nerol. It further includes vitamin C, vitamin A, calcium and zinc by giving the plant even a high nutritional value.

Officinal sage *(Salvia officinalis* L.): the used plant portion is the leaf. From the botanical point of view, it is a perennial and evergreen shrub formed by many dense leaves having a colour which can vary from grey to green, belonging to the *Laminaceae* family, originally from Southern Europe. The flowers appear at the apex of the stem in spring-summer and they are generally quite intense blue-purple. Since ancient times used as panacea by the Greeks, Romans and Egyptians. *"In nomen omen":* already from its name the beneficial consideration can be deduced that was attributed to this plant: Salvia from *salvus* (healthy) and *officinalis* (officinal). The Sage is one of the traditional remedies with stimulating, antibacterial, anti-stress, digestive, antispasmodic, antiperspirant, hypoglycemic properties. The infusion of Sage was used as expectorant for bronchitis and other respiratory disorders, for digestion and states of fatigue in general. The gargles with Save infusion instead to soothe tonsillitis, inflammation of the throat and the mouth, stomatitis and gingivitis. As far as the active components are concerned, the plant includes 1-2.8% of essential oil (produced in the summer periods), about 35% thereof is constituted by thujone (α- and β-thujone); the remaining part of oil is constituted by other terpene substances and by a minimum portion of mono and sesquiterpenes. Apart from oil, the plant is very rich in polyphenolic compounds (over 160), in particular the rosmarinic, caffeic, chlorogenic and labiatic acid. Tannins, flavonoids and triterpenes are very represented as well.

Rosmarinic acid: compounds of phenolic nature, deriving from the caffeic acid, identified for the first time in Rosmarin, from which it takes the name. It is the main constituent of the phytocomplex of many plants of the *Labiatae* family (thereto even Rosmarin belongs), where it is synthetized as metabolite for defence purposes. Pharmacokinetic studies showed that the rosmarinic acid has excellent bioavailability, both when taken orally and when administered with formulations for topical use. In particular, computational analyses estimated that the absorption of the rosmarinic acid, when taken orally, is equal to about 60%. Even the absorption at cutaneous level was studied, and through ex vivo study on intact human skin it was seen that the rosmarinic acid accumulates in the epidermis, whereas the penetration at hydrophilic level of derma is little (the rosmarinic acid is relatively insoluble in water). Emulsions of oil in water containing olive oil inside thereof facilitate the accumulation of rosmarinic acid, probably due to enhancers which facilitate the penetration thereof. The 2-month application of a cream containing rosmarinic acid did not show adverse effects on the skin suffering from atopic dermatitis; the use then in topical formulations is to be considered safe. Hypericum *(Hypericum perforatum* L.): the used plant portion is the flower. From the botanical point of view, the plant is native from Europe, Northern Africa and Western Asia, which nowadays can be defined ubiquitous considering its widespread throughout much of the world. It appears like a perennial and evergreen plant belonging to the botanic family of *Clusiacee* (or *Guttiferae).* The name of the species at issue, that is *perforatum,* derives from the Latin *perforo=* to perforate, and designates the several translucent glands present on the leaf blade, which make it to appear pitted. Thanks to this particular feature it is easily recognizable even when is it not flowering. The leaves are opposite on the stem, ovate-lanceolate in shape and bright green coloured. As said, they are covered with oily vesicles. In the margins there are small black points, consisting of glandular structures containing hypericin, the active principle of Hypericum. It is a red pigment contained in greater quantities in flowers. The flowers are yellow-gold coloured, formed by 5 very delicate petals, up to twice as long as the sepals. They can be found gathered in multiflorous corymbs. One of the most widespread uses of Hypericum is the external one, through its famous red oil, called, indeed Hypericum oil (oleolito). It is considered the natural remedy par excellence for all possible skin injuries, primarily burns, but also sores and wounds of different type, thanks to its capability of accelerating the healing process of, even serious, injuries, without causing wrinkling of the skin. It is also recommended as natural remedy for all skin lesions, such as excoriations, abrasions and rashes. It is used even to perform massages and frictions. It soothes pain caused by bruises and speeds up healing, by facilitating the resorption of hematoma and by reducing swelling. By oral route, instead, it is traditionally used for pathologies linked to the emotional state, for anxious, nervous and antidepressive states. The main constituents are hyperforin (2-4.5%) and hypericin (0.10-0.15%). Hypericin is a naphthodiantrone, an anthraquinone derivative, which constitutes one of the main chemical compounds isolated from plants of genus *Hypericum;* it is present in all anatomical parts except the roots. Hypericin appears like a bright red pigment (also called Hypericum red) which gives the characteristic red colour to the oleolite. Other phytoconstituents of the Hypericum are the flavonoids (hyperoside, rutin, quercetin, kampferol, catechins), tannins, caffeic acid, proanthocyanidins and a small amount of essential oil (0.05-0.9%), which also contains a minimal portion of β-caryophyllene. Under the term active principle in the present invention, one relates to a substance which has a certain biological activity, then all substances provided with therapeutical effect such as the drugs or beneficial such as vitamins and probiotics.

### SUMMARY OF THE INVENTION

The authors of the present invention have surprisingly found that a composition, comprising β-caryophyllene or oleoresin of *Copaifera officinalis* containing it, boswellic acids or resin extracts of *Boswellia serrata* containing it and escin or extracts of *Aesculus hippocastanum* containing it, has revealed to be particularly effective in offering relief in painful and inflammatory states of traumatic or rheumatic nature affecting joints, muscles, tendons and ligaments. The present invention allows to obtain at the same time a pain-relieving, anti-oedema effect improving the microcirculation, anti-inflammatory and muscle relaxant effect, but even an antioxidant and thermal effect.

In particular, the Authors have demonstrated that the Swiss stone pine essential oil and the Boswellia mixtures, copaiba oil and Escin, both in phytosomal form and in free form have an anti-inflammatory effect on human chondrocytes treated with IL-1β, by reducing in particular the production of MMP-13.

The mixture formulation with the active components in vehiculated form increases the effectiveness of the inflammatory effect. In particular, the mixture in phytosomal form has a greater effectiveness of about 30% than the corresponding mix in free form in inhibiting the release of MMP-13. Moreover, as to the combinatory effect, the Swiss stone pine essential oil and the phytosomal mixture show a synergic effect in reducing the production of MMP-13.

Moreover, the Authors have demonstrated that all tested mixtures have a cytotoxic effect on cells SW1353 when they are used at high concentrations (1 - 0.5 mg/mL). Below the concentration of 0.25 mg/mL the Swiss stone pine essential oil and the mixture formulated in phytosomal form do not result to be harmful, whereas the mixture consisting of the products in free form resulted to be more toxic, by reducing the cell viability up to a concentration of 0.031 mg/mL.

The above ingredients for the purpose of the present invention are primary active ingredients, and they are states associated to act on three different molecular targets:
- the endocannabinoid system,
- the arachidonic acid cascade,
- the glucocorticoid receptors.

In a preferred embodiment the composition of the invention conjugates plant extracts of the medicinal tradition (for example Common comfrey, Hypericum, Devil's claw) with vegetable extracts having high functional and technological value (for example β-caryophyllene from copaiba oil, Boswellia, escin).

In particular, the boswellic acids, β-caryophyllene from copaiba oil and escin are vehiculated in carrier systems (phytosomes and nanosomes) to increase the deep absorption of the active molecules and consequently the anti-inflammatory and analgesic effectiveness.

Therefore, the present invention firstly relates to a composition for topical use, comprising β-caryophyllene or oleoresin of *Copaifera officinalis* containing it, escin (or extracts of *Aesculus hippocastanum* containing it) and boswellic acids or resin extracts of *Boswellia serrata* containing boswellic acids, characterized in that said escin or extracts of *Aesculus hippocastanum* containing it and said boswellic acids or resin extracts of *Boswellia serrata* are in phytosomal form, the composition further comprising Swiss stone pine essential oil.

The present invention secondly relates to the composition as described in the present invention, for use in the treatment of painful and inflammatory states of traumatic or rheumatic nature affecting joints, muscles, tendons and ligaments.

Additional advantages and/or embodiments of the present invention will be evident from the following detailed description.

### DETAILED DESCRIPtioN OF FIGURES

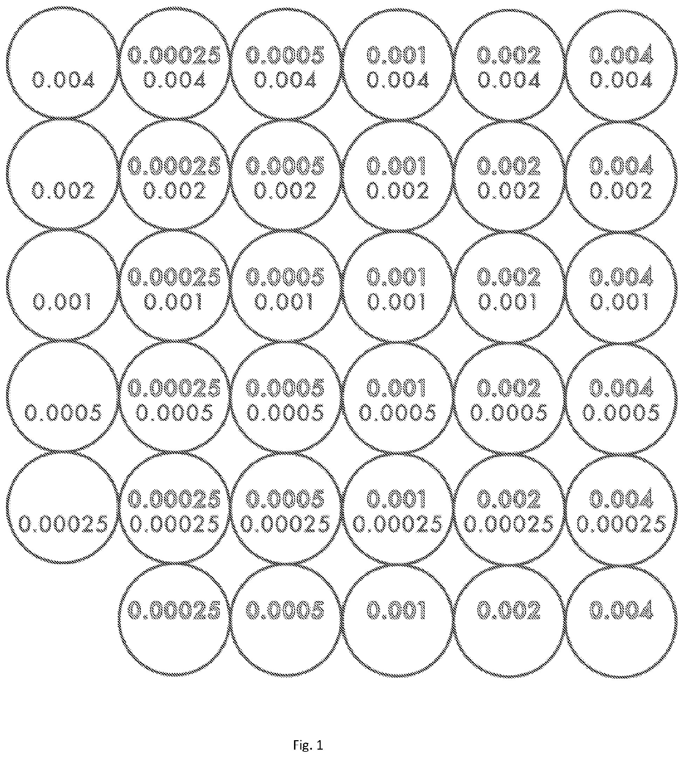

**Figure** 1 - **Synergy test scheme.** Each well, represented by a black circle, includes a unique combination of different concentrations of (brown) phytosomal mixture and (green) Swiss stone pine essential oil. The concentration values are shown in mg/mL.

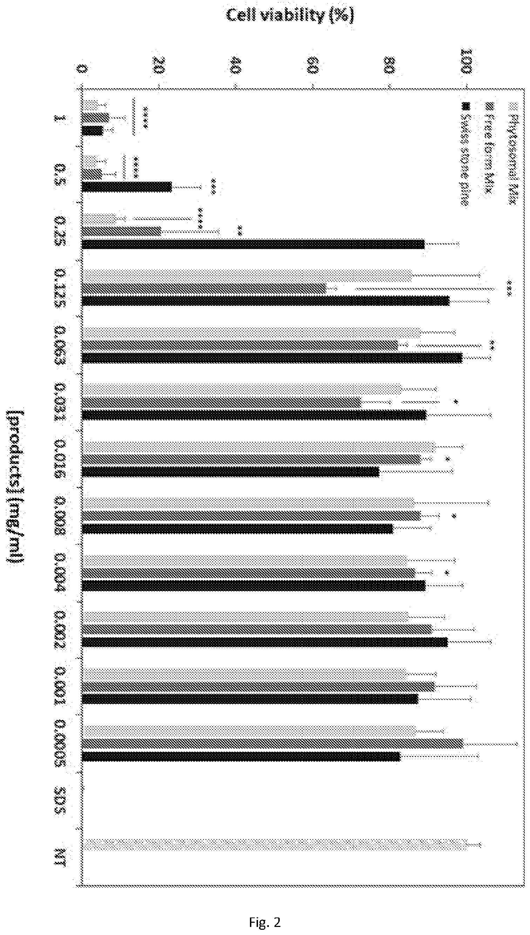

**Figure 2** **- Cell viability assay.** The cells SW1353 were treated with different concentrations of products for 24 h. The cell viability was determined by measuring the absorbance at 595 nm after reaction with MTT. The viability values are expressed as absorbance percentage of the not treated cells. SDS 1 mg/ml was used as positive control. The statistical analysis was performed by two-tailed test-t: * = p < 0.05; ** = p < 0.01; *** = p < 0.001; **** = p < 0.0001.

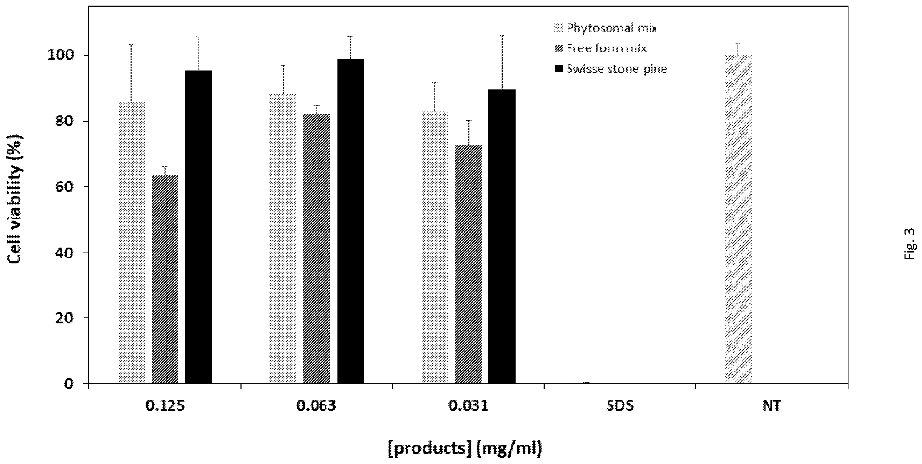

**Figure 3** **- Cell viability assay.** Detail display of the three concentrations wherein the Mix in phytosomal form does not result to be toxic whereas the free form has still relevant toxicity. The cells SW1353 were treated with different concentrations of the products for 24 h. The cell viability was determined by measuring the absorbance at 595 nm after reaction with MTT. The viability values are expressed as absorbance percentage of the not treated cells. SDS 1 mg/ml was used as positive control.

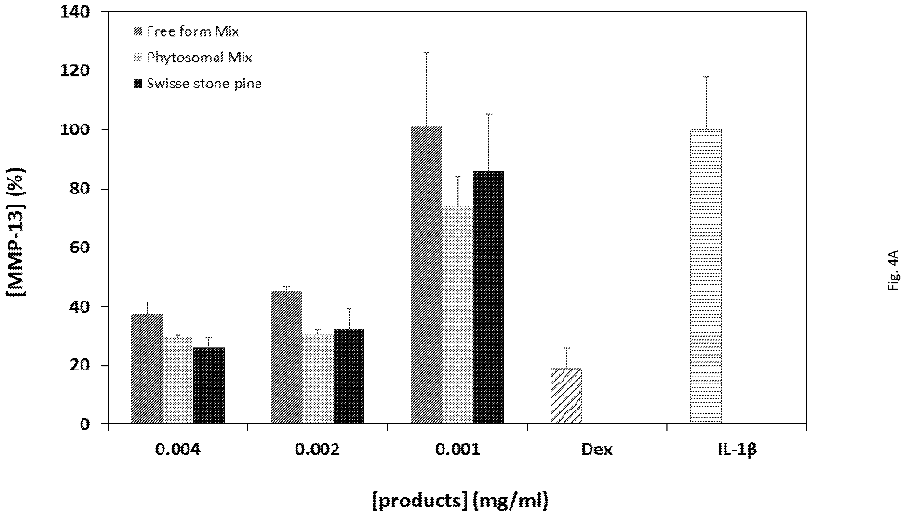

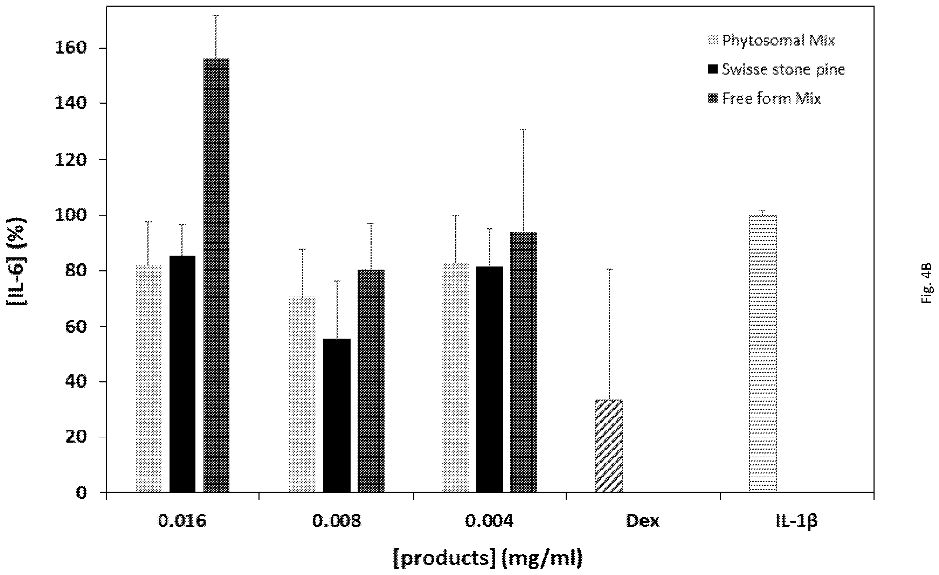

**Figure 4** **- Anti-inflammatory activity.** Production of MMP-13 (A) or IL-6 (B) with the three products tested singularly. The values are expressed as percentage of the respective cytokine produced in the cells treated with IL-1β only. As positive control, the anti-inflammatory drug Dexamethasone at the concentration of 0.1 mg/mL was used.

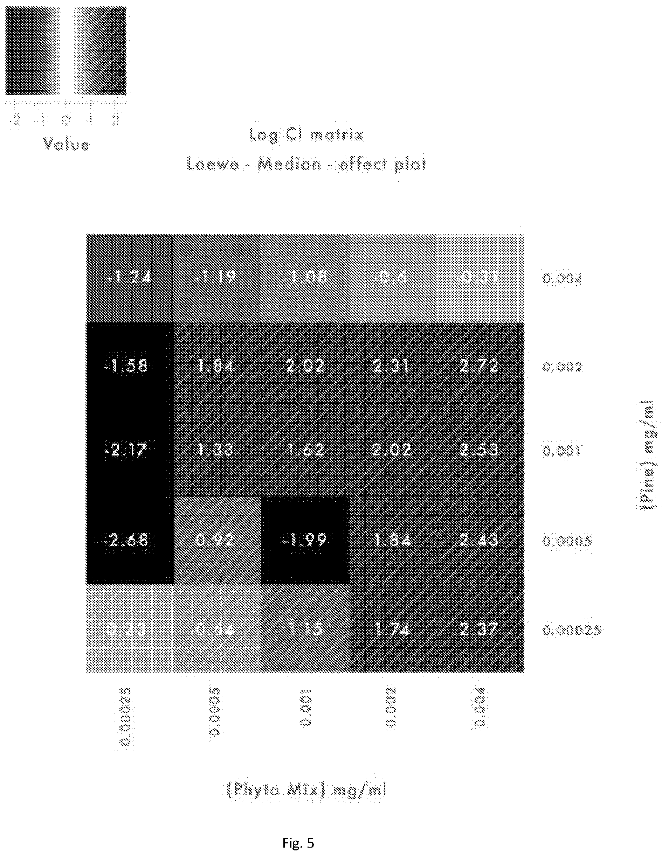

**Figure 5** **- Combination of Swiss stone pine essential oil and phytosomal mixture.** The values of logCl were calculated with software SiCoDEA (https://sicodea.shinyapps.io/shiny/), by using the Loewe additivity model. The logCl negative values, designating synergy, are represented in blue shades; the logCl positive values, designating antagonism, are represented in red shades.

### DETAILED DESCRIPTION

The Authors of the present invention have surprisingly found that a composition for topical use, comprising β-caryophyllene or oleoresin of *Copaifera officinalis* containing it, escin or extracts of *Aesculus hippocastanum* containing it and boswellic acids or resin extracts of *Boswellia serrata* containing it in form of a phytosome, has revealed to be particularly effective in offering relief in painful and inflammatory states of traumatic or rheumatic nature affecting joints, muscles, tendons and ligaments.

The present invention relates to a composition for topical use comprising β-caryophyllene or oleoresin of *Copaifera officinalis* containing it, escin or extracts of *Aesculus hippocastanum* containing it, boswellic acids or resin extracts of *Boswellia serrata* containing it and escin, characterized in that said boswellic acids or resin extracts of *Boswellia serrata* and said escin (or extracts of *Aesculus hippocastanum* containing it) are in phytosomal form, the composition further comprising Swiss stone pine essential oil.

In the present invention, β-caryophyllene relates to the sesquiterpene main component of oleoresin extracted from the trunk of *Copaifera officinalis* (Jacq.) L.

Resin extract of *Boswellia serrata* relates to the extracts obtained from gum resin obtained by incision of the bark. The collection is performed by producing oval decortications on the branches by using a kind of chisel sharpened on one side to decorticate the branches and not sharpened on the other side to collect the resin.

In the present invention, under escin one relates to the mixture di triterpene saponins isolated from the Horse Chestnut seed *(Aesculus hippocastanum).*

In order to obtain the Swiss stone pine essential oil, the twigs of *Pinus cembra* L are used.

In an embodiment, the composition further comprises one or more of the following substances: menthyl nicotinate, menthol, Devil's claw extract, Comfrey extract, Holy basil extract, Officinal sage extract, Eucalyptus essential oil, Hyypericum oil.

In a preferred embodiment, said composition comprises β-caryophyllene or oleoresin of *Copaifera officinalis* containing it, boswellic acids or resin extracts of *Boswellia serrata* containing it, escin or extracts of *Aesculus hippocastanum* containing it, Swiss stone pine essential oil, menthyl nicotinate, menthol, Devil's claw extract, Comfrey extract, Holy basil extract, Officinal sage extract, Eucalyptus essential oil, Hypericum oil.

The ingredients β-caryophyllene or oleoresin of *Copaifera officinalis* containing it, boswellic acids or resin extracts of *Boswellia serrata* containing it, escin or extracts of *Aesculus hippocastanum* containing it described herein represent, in an embodiment of the present invention, the main active principles.

The ingredients Swiss stone pine essential oil, menthyl nicotinate, menthol, Devil's claw extract, Comfrey extract, Holy basil extract, Officinal sage extract, Eucalyptus essential oil, Hypericum oil described herein represent in an embodiment of the present invention the secondary active principles.

In an embodiment, said Boswellia serrata resin extracts *Boswellia serrata* containing boswellic acids are in phytosomal form dispersed in natural soybean phospholipids.

In an embodiment, said escin is in form of a phytosome, in a form complexed with β-sitosterol and phospholipids.

In an additional embodiment, said Copaiba oil is in oleoresin form and/or in nanosomal and/or phytosomal and/or liposomal form. In a preferred embodiment, said Copaiba oil is present both in form of oleoresin and in nanosomial form. The nanosome represents a delivery system based on nanovesicles (liposomes having sizes 150-300 nm) which allow to incapsulate an active principle with high effectiveness and to release then the incapsulated compound. Such technology allows to guarantee greater stability of the extract, to increase bioavailability and the effectiveness thanks to its capability of crossing the biological membranes.

In an embodiment, said Holy basil extract and said Officinal sage extract are provided together in form of one single phytocompound, preferably BIOSTERINE^{®}.

In an embodiment, the composition of the present invention comprises:
- β-caryophyllene or oleoresin of *Copaifera officinalis* containing it in a range from 0.01% to 10% by weight with respect to the total weight of the composition, preferably from 0.01 to 3.0%, and/or
- boswellic acids or resin extracts of *Boswellia serrata* containing it in a range from 0.01% to 10% by weight with respect to the total weight of the composition, preferably from 0.01 to 3.0%, and/or
- escin or extracts of *Aesculus hippocastanum* containing it in a range from 0.01% to 10% by weight with respect to the total weight of the composition, preferably from 0.01 to 3.0%, and/or

In an embodiment, said Swiss stone pine essential oil is present in a range from 0.01% to 10% by weight with respect to the total weight of the composition, preferably from 0.01 to 3.0%.

In an additional embodiment, the composition of the invention comprises:
- β-caryophyllene or oleoresin of *Copaifera officinalis* containing it in a range from 0.01% to 10% by weight with respect to the total weight of the composition, preferably from 0.01 to 3.0%, and/or
- boswellic acids or resin extracts of *Boswellia serrata* containing it in a range from 0.01% to 10% by weight with respect to the total weight of the composition, preferably from 0.01 to 3.0%, and/or
- escin or extracts of *Aesculus hippocastanum* containing it in a range from 0.01% to 10% by weight with respect to the total weight of the composition, preferably from 0.01 to 3.0%, and/or
- Swiss stone pine essential oil in a range from 0.01% to 10% by weight with respect to the total weight of the composition, preferably from 0.01 to 3.0%, and/or
- menthyl nicotinate in a range from 0.01% to 5% by weight with respect to the total weight of the composition, preferably from 0.01 to 3.0%, and/or
- menthol in a range from 0.01% to 3% by weight with respect to the total weight of the composition, preferably from 0.01 to 1.5%, and/or
- Devil's claw extract from 0.01% to 10% by weight with respect to the total weight of the composition, preferably from 0.01 to 3.0%, and/or
- Comfrey extract from 0.01% to 15% by weight with respect to the total weight of the composition, preferably from 0.01 al 5,0%, and/or
- Holy basil extract from 0.01% to 10% by weight with respect to the total weight of the composition, preferably from 0.01 to 3.0%, and/or
- Officinal sage extract from 0.01% to 10% by weight with respect to the total weight of the composition, preferably from 0.01 to 3.0%, and/or
- Eucalyptus essential oil from 0.01% to 5% by weight with respect to the total weight of the composition, preferably from 0.01 to 1.5%, and/or
- Hypericum oil from 0.01% to 10% by weight with respect to the total weight of the composition, preferably from 0.01 to 3.0%.

To the purpose of the present invention, said Devil's claw extract is obtained from the root of *Harpagophytum procumbens* L. (Burch.) and/or, said Comfrey extract is obtained from the root of *Symphytum officinale* L. and/or, said Holy basil extract is obtained from the leaves of *Ocimum sanctum* L., and/or said Officinal sage extract is obtained from the leaves of *Salvia officinalis* L and/or., said Eucalyptus essential oil is obtained from the leaves of *Eucalyptus globulus* L. and/or, said Hypericum oil is obtained from the flowers of *Hypericum perforatum L.*

In an embodiment, the composition of the invention further comprises an emulsifying agent. Said emulsifying agent is any emulsifying agent known to the person skilled in the art suitable to the compositions described in the present invention. In particular, said emulsifying agent is an emulsifying system comprising glyceryl stearate, cetearyl alcohol, hydrolyzed palmitoyl potassium and wheat proteins.

In an embodiment, the composition of the invention further comprises a preservative agent. Said preservative agent is any emulsifying agent known to the person skilled in the art suitable to the compositions described in the present invention. In particular, said preservative agent is a preservative system comprising 1,2 hexanediol, caprylyl glycol and hydroxyacetophenone.

In an embodiment, the composition of the present invention can be in form of a cream, ointment, pomade, lotion, tonic, gel, paste, foam, hydrophobic gel, hydrophilic gel, emulsion, suspension or liquid. Depending upon the type of formulation, the composition can further comprise one or more excipients and/or carriers. For lipophilic ointments, the excipients can be selected from: solid, semi-solid and/or liquid paraffins, vegetable oils, waxes, liquid silicones, which generally have occlusive or protective properties. It is also possible to add surfactants such as sorbitan esters, lanolin alcohols, fatty alcohols, fatty acid sulphates, fatty acid esters and/or polysorbates to obtain an ointment that emulsifies in waters.

On the contrary, as to the composition of the present invention in the form of hydrophilic ointment, suitable excipients to be added will be mixtures of polyethylenglycoles.

With reference to a possible embodiment wherein the composition described by the present invention is in form of cream, since cream is a multi-phase preparation consisting of a lipophilic phase and a hydrophilic phase, the addition of emulsifying agents is fundamental. In case these are hydrophobic creams, the emulsifying agents which can be added are wool alcohols, sorbitan esters and/or monoglycerides, while with reference to hydrophilic creams the emulsifying agents will be sodium soaps, polysorbates and/or fatty acid sulphates.

As to compositions formulated as hydrophilic gels, the addition of hydrophilic macromolecules results to be essential, such as sodium alginate, agar-agar, gums, gelatin, starch, cellulose derivatives and/or carboxy-vinyl-polymers.

In case of hydrophobic gel, the excipients which can be included are: micronized silica, hydrogenated castor oil, stearyl-ammonium hettorite, zinc stearates, calcium stearates and/or aluminium stearates.

Other excipients can be hydrating substances such as collagen, elastin, various protein hydrolysates, nucleic acid hydrolysates, natural moisturizing factor; or absorption promoters, such as DMSO, fatty acids.

All embodiments described up to now could be prepared by means of any one of the known methods used in the cosmetic field.

According to an embodiment, said composition is a pharmaceutical, cosmetic composition, a medical device.

A medical device, in the present invention, corresponds to a medical device according to any one of the classes described in the Regulation (UE) 2017/745 about medical devices (which comprises also substances and not only "devices" in the mechanical meaning of the term.)

The composition shown in the present description can be used as medicament.

The present invention also relates to the composition as described in the present disclosure, for use in the treatment of painful and inflammatory states of traumatic or rheumatic nature affecting joints, muscles, tendons and ligaments.

In an embodiment, said states of traumatic or rheumatic nature are arthritis, contractures, injuries, tendinitis, strains, tears, contusions, sprains and hematomas.

The present invention also relates to a method for the treatment of painful and inflammatory states of traumatic or rheumatic nature affecting joints, muscles, tendons and ligaments, which provide the topical administration of the composition according to any one of the embodiments of the invention, on a subject requiring it.

The present invention further relates to the use of the composition according to any one of the embodiments, for manufacturing a medicament for the treatment of painful and inflammatory states of traumatic or rheumatic nature affecting joints, muscles, tendons and ligaments.

In any part of the present description and claims, the term comprising can be replaced by "consisting of".

Herebelow examples are reported having the purpose of better illustrating the methods disclosed in the present description, such examples are not to be considered in any way a limitation of the preceding description and of the following claims.

### EXAMPLES

In vitro anti-inflammatory tests of a formulation for topical use, comprising active principles in vehiculated form, were performed.

The product consists of a mixture of phytosomal Boswellia, nanosomal Copaiba oil and phytosomal Escin, in ratio 1:1:0.5; its properties were compared to those of a mixture consisting of the same active principles in the same proportions but in free form.

During the execution of the tests, to obtain comparable data, two mixtures were tested by standardizing the concentrations based upon the net content of active principles.

In the anti-inflammatory assays in addition the properties of a Swiss stone pine essential oil were investigated, since it is a compound of interest for reinforcing the above-mentioned formula.

In the experimental plan the first step was the evaluation of the cytotoxic effect of each compound on the cell line of human chondrocytes. Subsequently, the capability of the single products to reduce the production of Metalloprotease of matrix 13 (MMP-13) and Interleukin 6 (IL-6) in the cell line of human chondrocytes (SW1353), induced in an inflammatory state by means of treatment with Interleukin 1-beta (IL-1β), was tested. Such markers, in fact, are important indices of the inflammatory state.
IL-6 is an inflammatory cytokine involved in the process of degeneration of cartilage and in inflammatory pain, whereas MMPs are a group of zinc-dependent endopeptidases which, if in excess, cause the degeneration of the extracellular matrix of the cartilage.
After the tests with the single compounds a combinatory anti-inflammatory test was performed at last, by mixing in different ratios the mix in phytosomal form with the Swiss stone pine essential oil, to determine the possible synergic effect of the two formulates.

### Materials and methods

### Cell lines and culture conditions

The experiments were performed on a cell line of human chondrocytes (SW1353). If not specified, the cells were cultivated at 37°C in absence of CO2 in medium L-15 (Leibovitz), integrated with 10% of fetal bovine serum (FBS).

### Cell viability test (MTT assay)

The cells SW1353 were diluted at the concentration of 10 cells/mL in Dulbecco's Modified Eagle Medium (DMEM) supplemented with 10% FBS. 100 µL of this suspension were aliquoted in a sterile 96-well plate, incubated overnight at 37°C and 5% CO2. At the end of incubation, the medium was removed and replaced with 100 µL of complete DMEM integrated with 1% FBS and containing the products at the following concentrations (in mg/mL): 1 - 0.5 - 0.25 - 0.125 - 0.063 - 0.031 - 0.016 - 0.008 - 0.004 - 0.002 - 0.001 - 0.0005. As positive control sodium dodecyl sulphate (SDS) at the concentration of 1 mg/ml was used. The cells were incubated for 24 hours at 37°C and 5% CO2. At the end of the treatment, to each well the substrate 3-(4,5-dimethylthiazol-2-yl)-2,5-diphenyltetrazolium bromide (MTT) at the final concentration of 0.45 mg/mL was added and the plate was incubated for further 2 hours at 37°C and 5% CO2.

At the end of incubation, the crystals of reduced MTT were solubilized by removing the medium and by adding 100 µL of DMSO to each well. At last, the absorbance at 595 nm of each sample was measured, by means of a plate reader iMark Microplate Reader (Biorad). The cell viability is expressed as percentage of absorbance at 595 nm of the not treated cells (NT).

### Ant-inflammatory activity test

The cells SW1353 were diluted at a concentration of 10 cells/ml in DMEM with 10% FBS and 1 mL of this suspension was aliquoted in each well of a 48-multiwell plate, incubated at 37°C and 5% CO2 for 72h. At the end of incubation, the medium was replaced by 500 µL of complete DMEM without FBS, containing 5 ng/ml of IL-1β alone or with each product at the following concentrations (in mg/mL): 0.002 - 0.001 - 0.0005. As positive control, the anti-inflammatory drug Dexamethasone, at the concentration of 0.1 mg/ml, was used. The treatment was performed for 24 hours, at the end thereof aliquots of 100 µL of the culture medium were collected and then analysed through ELISA test, to quantify the production of MMP-13 and IL-6.

### Synergies

The synergic anti-inflammatory treatment was performed by using the mixture in phytosomal form and the Swiss stone pine essential oil in 25 different combinations. The test was performed according to the "chessboard method" which allows to evaluate the combined effect of two compounds in mixture, by crossing serial dilutions of the same (Figure 1). In particular, 5 concentrations of both compounds were crossed: 0.004 - 0.002 - 0.001 - 0.0005 - 0.00025 mg/mL.

The anti-inflammatory test was performed under the same conditions described in the preceding paragraph, by measuring the production of MMP-13, since most representative marker of the anti-inflammatory activity.

The combinatory effect of the different concentrations of the two products was evaluated by using the software "Single and Combined Drug Effect Analysis" (SiCoDEA). For each combination the Combination Index (CI) according to the Loewe additivity model was calculated, by using the equation of the median effect as dose-response curve.

### ELISA

The detection of MMP-13 and IL-6 was performed on 100 µL of each sample, by following the protocols of kits provided by the producers (Abcam or Merck). The quantification was performed by setting up calibration lines with different concentrations of the two inflammatory cytokines.

The anti-inflammatory effect is expressed as percentage of the concentration of MMP-13 or IL-6 with respect to the cells treated only with IL-1β.

### Results

### Cell viability

The three products have a cytotoxic effect on the cells SW1353 when they are used at high concentrations (1 - 0.5 mg/mL). Below the concentration of 0.25 mg/mL the Swiss stone pine essential oil and the mixture formulated in phytosomal form are no more harmful, whereas the mixture composed of the products in free form resulted to be more toxic: in fact, it reduces the cell viability until a concentration of 0.031 mg/mL (Figures 2 and 3). For this reason, for the subsequent anti-inflammatory tests, the products were used at lower concentrations than 0.031 mg/mL.

### Anti-inflammatory activity

The cells SW1353 were treated with IL-1β to induce the expression of the proinflammatory markers MMP-13 and IL-6. The different compounds were evaluated based upon their capability of inhibiting this process.

In the first test phase, the products were evaluated singularly at three different concentrations.

Since the effect on the release of the marker IL-6 resulted to be lower (not shown) for the test on this marker, concentrations of 0.016, 0.008 and 0.004 mg/mL were used. Given the responsiveness of MMP-13, in this case the products were used at the concentrations of 0.004, 0.002 and 0.001 mg/mL.

Based upon the obtained data, one observed that all products are capable of reducing effectively the production of MMP-13 by the cells SW1353 treated with IL-1β. The effect seems to be dependant from concentration, but in all cases the phytosomal mixture and the Swiss stone pine essential oil have a higher anti-inflammatory effect than that of the mixture of compounds in free form. At higher concentrations, one obtains a decrease in the production of MMP-13 of almost 80% with respect to the control treated only with IL-1β, an effect comparable to that of the anti-inflammatory drug Dexamethasone (Figure 4A).

The effect of the compounds on IL-6 is lower: in this case the inhibition of the production of the proinflammatory cytokine is about 20-40%. However, even in this case, the phytosomal mixture and the Swiss stone pine essential oil seem to have a greater effect than that of the mixture of compounds in free form, which at high concentrations (0.016 mg/mL) seems to have an effect of inducing the production of IL-6 (Figure 4B).

Based upon these data, the phytosomal mixture and the Swiss stone pine essential oil were used in combination in different concentrations to verify the synergic effect thereof on the production of MMP-13 in cells SW1353 treated with IL-1β.

The effect of the simultaneous use of the two compounds was evaluated by calculating the Combination Index (CI) based upon the Loewe additivity model. According to this principle, a Cl of 1 corresponds to a usual additive effect, a Cl ranging from 0 and 1 designates synergy between the two compounds, and CI higher than 1 designates antagonism.

The results are shown in figure 5. In order to make easier the interpretation, the logarithm values of Cl (logCI) are shown, so that: logCI negative = synergy; loCI 0 = additive effect; logCl positive = antagonism.

As it can be observed, the two products show a clear synergic effect, especially when low concentrations of phytosomal mixture are used (0.00025 mg/mL). The effect seems to increase as the Swiss stone pine essential oil concentration decreases, the maximum being obtained when the latter is used at the concentration of 0.0005 mg/mL (logCl = - 2.68).

A clear synergic effect is observed even when the Swiss stone pine essential oil is used at high concentrations (0.004 mg/mL), which increases as the concentration of phytosomal mixture decreases. However, the effect is lower than the one observed at low concentrations of both compounds.

The phytosomal mixture and the Swiss stone pine essential oil, at last, operate synergically even when they are used at the concentrations of 0.001 and 0.0005 mg/mL, respectively.

### Conclusions

The experimentation has allowed to attribute a clear anti-inflammatory effect to the Swiss stone pine essential oil and to the mixtures of Boswellia, copaiba oil and Escin, both in phytosomal form and in free form. In particular, the three products seem to be capable of reducing effectively the production of the proinflammatory marker MMP-13, in human chondrocytes treated with IL-1β, whereas the effect on the production of IL-6 results to be lower. In both cases, however, the mixture formulation in phytosomal form seems to increase the effectiveness of the anti-inflammatory effect. In particular, the mixture in phytosomal form has a greater effectiveness of about 30% with respect to the corresponding mix in free form in inhibiting the release of MMP-13.

At last, the products Swiss stone pine essential oil and phytosomal mixture, when they are used in combination, show a synergic effect in reducing the production of MMP-13, especially when they are used at low concentrations. The higher effect is observed with the combination: Swiss stone pine = 0.0005 mg/mL + phytosomal mixture = 0.00025 mg/mL.

**Table 1: example of primary and secondary active ingredients and relative functions in a composition of the invention.**

| | **ACTIVE INGREDIENTS** | **FUNCTION** |
|---|---|---|
| Primary active ingredients: | NANOSOMAL COPAIBA OIL and COPAIBA OIL | PAIN RELIEVER |
| | PHYTOSOMAL BOSWELLLIA | ANTI-INFLAMMATORY |
| | PHYTOSOMAL ESCIN | ANTI-OEDEMA MICROCIRCULATION |
| | SWISS STONE PINE ESSENTIAL OIL | ANTI-INFLAMMATORY MIORELAXING |
| Secondary active ingredients: | MENTHYL NICOTINATE MENTHOL | MICROCIRCULATION ANTI-OEDEMA ANALGESIC THERMAL |
| | DEVIL'S CLAY | ANTI-INFLAMMATORY |
| | COMFREY | ANTI-INFLAMMATORY |
| | BIOSTERINE^{®} | ANTI-INFLAMMATORY ANALGESIC |
| | EUCALYPTUS ESSENTIAL OIL | ANALGESIC |
| | HYPERICUM | ANTI-INFLAMMATORY ANTIOXIDANT |

Emulsifying system: glyceryl stearate, cetearyl alcohol, potassium palmitoyl hydrolyzed wheat protein.

Preservative system: 1,2-hexanediol, caprylyl glycol, hydroxyacetophenone.

## Claims

1. A composition for topical use comprising β-caryophyllene or oleoresin of *Copaifera officinalis* containing it, escin or extracts of *Aesculus hippocastanum* containing it and boswellic acids or resin extracts of *Boswellia serrata* containing it, **characterized in that** said escin or extracts of *Aesculus hippocastanum* and said boswellic acids or resin extracts of *Boswellia serrata* are in phytosomal form, the composition further comprising Swiss stone pine essential oil.

2. The composition according to claim 1, further comprising one or more of the following substances: menthyl nicotinate, menthol, Devil's claw extract, Comfrey extract, Holy basil extract, Officinal sage extract, Eucalyptus essential oil, Hypericum oil.

3. The composition according to any one of claims 1 or 2, comprising: β-caryophyllene or oleoresin of *Copaifera officinalis* containing it, boswellic acids or resin extracts of *Boswellia serrata* containing it, escin or extracts of *Aesculus hippocastanum* containing it, Swiss stone pine essential oil, menthyl nicotinate, menthol, Devil's claw extract, Comfrey extract, Holy basil extract, Officinal sage extract, Eucalyptus essential oil, Hypericum oil.

4. The composition according to any one of claims 1 to 3, wherein said *Boswellia serrata* resin extracts containing boswellic acids are in phytosomal form dispersed in natural soybean phospholipids.

5. The composition according to any one of claims 1 to 5, wherein said escin or extracts of *Aesculus hippocastanum* containing it in form of a phytosome is in a form complexed with β-sitosterol and phospholipids.

6. The composition according to any one of claims 1 to 5, wherein said β-caryophyllene or oleoresin of *Copaifera officinalis* containing it is in oleoresin form and/or in nanosomal and/or phytosomal and/or liposomal form.

7. The composition according to any one of claims 1 to 6, comprising:
- said β-caryophyllene or oleoresin of *Copaifera officinalis* containing is in a range from 0.01% to 10% by weight with respect to the total weight of the composition, preferably from 0.01 to 3.0%, and/or
- said boswellic acids or resin extracts of *Boswellia serrata* containing it are present in a range from 0.01% to 10% by weight with respect to the total weight of the composition, preferably from 0.01 to 3.0%, and/or
- said escin or extracts of *Aesculus hippocastanum* containing it is present in a range from 0.01% to 10% by weight with respect to the total weight of the composition, preferably from 0.01 to 3.0%.

8. The composition according to any one of claims 1 to 7, wherein:
- said Swiss stone pine essential oil is present in a range from 0.01% to 10% by weight with respect to the total weight of the composition, preferably from 0.01 to 3.0%.

9. The composition according to any one of claims 1 to 8, in form of a cream, ointment, pomade, lotion, tonic, gel, paste, foam, hydrophobic gel, hydrophilic gel, emulsion, suspension or liquid.

10. The composition, according to any one of claims 1 to 9, for use in the treatment of painful and inflammatory states of traumatic or rheumatic nature affecting joints, muscles, tendons and ligaments.

11. The composition for use according to claim 10, wherein said states of traumatic or rheumatic nature are arthritis, contractures, injuries, tendinitis, strains, tears, contusions, sprains and hematomas.

## Patentansprüche

1. Zusammensetzung zur topischen Verwendung, umfassend β-Caryophyllen oder dieses enthaltendes Oleoresin aus *Copaifera officinalis*, Aescin oder dieses enthaltende Extrakte aus *Aesculus hippocastanum* und Boswelliasäuren oder diese enthaltende Harzextrakte aus *Boswellia serrata*, **dadurch gekennzeichnet, dass** das Aescin oder die Extrakte aus *Aesculus hippocastanum* und die Boswelliasäuren oder die Harzextrakte aus *Boswellia serrata* in phytosomaler Form vorliegen, die Zusammensetzung ferner umfassend ätherisches Zirbenöl.

2. Zusammensetzung nach Anspruch 1, ferner umfassend eine oder mehrere der folgenden Substanzen: Menthylnicotinat, Menthol, Teufelskrallenextrakt, Beinwellextrakt, Extrakt aus Heiligem Basilikum, Extrakt aus Echtem Salbei, ätherisches Eukalyptusöl, Johanniskrautöl.

3. Zusammensetzung nach einem der Ansprüche 1 oder 2, umfassend: β-Caryophyllen oder dieses enthaltendes Oleoresin aus *Copaifera officinalis*, Boswelliasäuren oder diese enthaltende Harzextrakte aus *Boswellia serrata*, Aescin oder dieses enthaltende Extrakte aus *Aesculus hippocastanum*, ätherisches Zirbenöl, Menthylnicotinat, Menthol, Teufelskrallenextrakt, Beinwellextrakt, Extrakt aus Heiligem Basilikum, Extrakt aus Echtem Salbei, ätherisches Eukalyptusöl, Hypericumöl.

4. Zusammensetzung nach einem der Ansprüche 1 bis 3, wobei die Harzextrakte aus *Boswellia serrata,* die Boswelliasäuren enthalten, in phytosomaler Form dispergiert in natürlichen Sojabohnenphospholipiden vorliegen.

5. Zusammensetzung nach einem der Ansprüche 1 bis 5, wobei das Aescin oder die dieses enthaltenden Extrakte aus *Aesculus hippocastanum* in Form eines Phytosoms in einer mit β-Sitosterol und Phospholipiden komplexierten Form vorliegen.

6. Zusammensetzung nach einem der Ansprüche 1 bis 5, wobei das β-Caryophyllen oder das dieses enthaltende Oleoresin aus *Copaifera officinalis* in Oleoresinform und/oder in nanosomaler und/oder phytosomaler und/oder liposomaler Form vorliegt.

7. Zusammensetzung nach einem der Ansprüche 1 bis 6, umfassend:
- Vorliegen des β-Caryophyllens oder des dies enthaltenden Oleoresins aus *Copaifera officinalis* in einem Bereich von 0,01 Gew.-% bis 10 Gew.-%, bezogen auf das Gesamtgewicht der Zusammensetzung, vorzugsweise von 0,01 bis 3,0 Gew.-%, und/oder
- Vorhandensein der Boswelliasäuren oder der diese enthaltenden Harzextrakte aus *Boswellia serrata* in einem Bereich von 0,01 Gew.-% bis 10 Gew.-%, bezogen auf das Gesamtgewicht der Zusammensetzung, vorzugsweise von 0,01 bis 3,0 Gew.-%, und/oder
- Vorhandensein des Aescins oder der dieses enthaltenden Extrakte aus *Aesculus hippocastanum* in einem Bereich von 0,01 Gew.-% bis 10 Gew.-%, bezogen auf das Gesamtgewicht der Zusammensetzung, vorzugsweise von 0,01 bis 3,0 Gew.-%.

8. Zusammensetzung nach einem der Ansprüche 1 bis 7, wobei:
- das ätherische Zirbenöl in einem Bereich von 0,01 Gew.-% bis 10 Gew.-%, bezogen auf das Gesamtgewicht der Zusammensetzung, vorzugsweise von 0,01 bis 3,0 Gew.-%, vorhanden ist.

9. Zusammensetzung nach einem der Ansprüche 1 bis 8 in Form einer Creme, einer Salbe, einer Pomade, einer Lotion, eines Tonikums, eines Gels, einer Paste, eines Schaums, eines hydrophoben Gels, eines hydrophilen Gels, einer Emulsion, einer Suspension oder einer Flüssigkeit.

10. Zusammensetzung nach einem der Ansprüche 1 bis 9 zur Verwendung bei der Behandlung von schmerzhaften und entzündlichen Zuständen traumatischer oder rheumatischer Art, die Gelenke, Muskeln, Sehnen und Bänder betreffen.

11. Zusammensetzung zur Verwendung nach Anspruch 10, wobei die Zustände traumatischer oder rheumatischer Art Arthritis, Kontrakturen, Verletzungen, Tendinitis, Zerrungen, Risse, Prellungen, Verstauchungen und Hämatome sind.

## Revendications

1. Composition à usage topique comprenant du β-caryophyllène ou de l'oléorésine de *Copaifera officinalis* en contenant, de l'escine ou des extraits d'*Aesculus hippocastanum* en contenant et des acides boswelliques ou des extraits de résine de *Boswellia serrata* en contenant, **caractérisée en ce que** ladite escine ou lesdits extraits d'*Aesculus hippocastanum* et lesdits acides boswelliques ou extraits de résine de *Boswellia serrata* sont sous forme phytosomale, la composition comprenant en outre de l'huile essentielle de pin cembro.

2. Composition selon la revendication 1, comprenant en outre une ou plusieurs des substances suivantes : nicotinate de menthyle, menthol, extrait de griffe du diable, extrait de consoude officinale, extrait de basilic sacré, extrait de sauge officinale, huile essentielle d'eucalyptus, huile de millepertuis.

3. Composition selon l'une quelconque des revendications 1 ou 2, comprenant : du β-caryophyllène ou de l'oléorésine de *Copaifera officinalis* en contenant, des acides boswelliques ou des extraits de résine de *Boswellia serrata* en contenant, de l'escine ou des extraits d'*Aesculus hippocastanum* en contenant, de l'huile essentielle de pin cembro, du nicotinate de menthyle, du menthol, de l'extrait de griffe du diable, de l'extrait de consoude officinale, de l'extrait de basilic sacré, de l'extrait de sauge officinale, de l'huile essentielle d'eucalyptus, de l'huile de millepertuis.

4. Composition selon l'une quelconque des revendications 1 à 3, dans laquelle lesdits extraits de résine de *Boswellia serrata* contenant des acides boswelliques sont sous forme phytosomale dispersés dans des phospholipides naturels de soja.

5. Composition selon l'une quelconque des revendications 1 à 5, dans laquelle ladite escine ou les extraits d'*Aesculus hippocastanum* en contenant sous forme de phytosome est sous une forme complexée avec du β-sitostérol et des phospholipides.

6. Composition selon l'une quelconque des revendications 1 à 5, dans laquelle ledit β-caryophyllène ou l'oléorésine de *Copaifera officinalis* en contenant est sous forme d'oléorésine et/ou sous forme nanosomale et/ou phytosomale et/ou liposomale.

7. Composition selon l'une quelconque des revendications 1 à 6, comprenant :
- ledit β-caryophyllène ou ladite oléorésine de *Copaifera officinalis* en contenant est compris dans une plage allant de 0,01 % à 10 % en poids par rapport au poids total de la composition, de préférence de 0,01 à 3,0 %, et/ou
- lesdits acides boswelliques ou extraits de résine de *Boswellia serrata* en contenant sont présents dans une plage de 0,01 % à 10 % en poids par rapport au poids total de la composition, de préférence de 0,01 à 3,0 %, et/ou
- ladite escine ou les extraits d'*Aesculus hippocastanum* en contenant est présente dans une plage de 0,01 % à 10 % en poids par rapport au poids total de la composition, de préférence de 0,01 à 3,0 %.

8. Composition selon l'une quelconque des revendications 1 à 7, dans laquelle :
- ladite huile essentielle de pin cembro est présente dans une plage de 0,01 % à 10 % en poids par rapport au poids total de la composition, de préférence de 0,01 à 3,0 %.

9. Composition selon l'une quelconque des revendications 1 à 8, sous forme de crème, d'onguent, de pommade, de lotion, de tonique, de gel, de pâte, de mousse, de gel hydrophobe, de gel hydrophile, d'émulsion, de suspension ou de liquide.

10. Composition, selon l'une quelconque des revendications 1 à 9, pour une utilisation dans le traitement des états douloureux et inflammatoires de nature traumatique ou rhumatismale affectant les articulations, les muscles, les tendons et les ligaments.

11. Composition pour utilisation selon la revendication 10, dans laquelle lesdits états de nature traumatique ou rhumatismale sont l'arthrite, les contractures, les lésions, les tendinites, les élongations, les déchirures, les contusions, les entorses et les hématomes.
